# EUROPEAN PATENT SPECIFICATION

(11) **EP 1 846 074 B1**
(45) Date of publication and mention of the grant of the patent: **22.01.2014**
(21) Application number: 05853125.2
(22) Date of filing: 07.12.2005
(51) Int. Cl.: A61L 29/12, A61M 25/00, A61M 25/10

(54) **NOVEL MICROFIBRILLAR REINFORCED POLYMER-POLYMER COMPOSITES FOR USE IN MEDICAL DEVICES**
NEUE MIKROFIBRILLÄRE VERSTÄRKTE POLYMER-POLYMER-VERBUNDSTOFFE ZUR VERWENDUNG IN MEDIZINISCHEN VORRICHTUNGEN
NOUVEAUX COMPOSITES POLYMERE-POLYMERE MICROFIBRILLAIRE RENFORCES DESTINES A DES DISPOSITIFS MEDICAUX

(30) Priority: 11.02.2005 US 55837
(43) Date of publication of application: 24.10.2007
(73) Proprietor: Boston Scientific Limited, Christ Church (BB)
(72) Inventor: ATANASOSKA, Liliana, Edina, Minnesota 55436 (US); HOLMAN, Thomas, J., Princeton, MN 55371 (US); SCHOENLE, Victor, B., Greenfield, Minnesota 55357 (US); WARNER, Robert, W., Woodbury, Minnesota 55125 (US); SCHEWE, Scott, Eden Prairie, Minnesota 55346 (US)
(74) Representative: Lang, Johannes
(86) International application number: PCT/US2005/044115
(87) International publication number: WO 2006/088537

(56) References cited:
- WO-A-01/34062
- US-A- 5 248 305
- US-A1- 2001 043 998
- FRIEDRICH: "direct electron microscopic observation of transcrystalline layers in microfibrillar reinforeced polymer-polymer composites" JOURNAL OF MATERIAL SCIENCE, vol. 37, 2002, pages 4299-4305, XP002393193 cited in the application
- FAKIROV S.: "microfibrillar reinforced composites from binary and ternary blends of polyesters and nylon 6" MACROMOLECULES, vol. 26, 1993, pages 5219-5226, XP002393194

## Description

### FILED OF THE INVENTION

The present invention relates to the field of medical devices, and to the use of microfibrillar polymer-polymer composites in such medical devices.

### BACKGROUND OF THE INVENTION

Microfibrillar polymer-polymer composites (MFCs) are those composites described in the art as having an isotropic matrix from a lower melting polymer reinforced by microfibrils of a higher melting polymer.

MFCs are created during manufacturing by drawing (fibrillization step) followed by melting of the lower-melting component during processing (isotropization step), with preservation of the microfibrils of the higher-melting component.

MFCs are discussed in Evstatiev, M., et al., Structure-Property Relationships of Injection- and Compression-Molded Microfibrillar-Reinforced PET/PA-6 Composites, Advances in Polymer Technology, Vol. 19, No. 4, pp. 249-259 (2000); Friedrich, K. et al., Direct electron microscopic observation of transcrystalline layers in microfibrillar reinforced polymer-polymer composites, Journal of Materials Science 37, pp. 4299-4305 (2002); Fakirov, S., et al., Crystallization in Partially Molten Oriented Blends of Polycondensates as Revealed by X-ray Studies, J. Macromol. Sci.-Physics, B40(5), pp. 935-957 (2001); Evstatiev, M., et al., PET/LDPE Microfibrillar Reinforced Polymer-Polymer Composites; Denchev, Z., et al., Nanostructured Composites Based on Polyethylene-Polyamide Blends. I. Preparation and Mechanical Behavior, Journal of Macromolecular Science Part B-Physics, Vol. B43, No. 1, pp. 143-162 (2004).

Balloon catheters generally comprise a catheter shaft with a balloon on the distal end of the shaft, and are used in a number of procedures, such as percutaneous transluminal coronary angioplasty (PTCA). In PTCA the balloon catheter is used to restore free flow in a clogged coronary vessel. The catheter is maneuvered through the patient's tortuous anatomy and into the patient's coronary anatomy until the balloon is properly positioned across the stenosis to be dilated. Once properly positioned, the balloon is inflated with liquid to reopen the coronary passageway.

The catheter shaft is typically provided with a relatively stiff proximal shaft section and a relatively flexible distal shaft section. The stiffened proximal shaft section provides greater push to the catheter which facilitates advancement over a guidewire. The stiffness can provide greater torqueability so that torque applied to the proximal end of the catheter extending outside of the patient results in rotation of the distal tip of the catheter. For overall catheter performance, it is desirable to balance proximal shaft stiffness against maintenance of a low profile and distal shaft flexibility.

Fibrils, such as those formed from liquid crystal polymers, have been employed in the manufacture of catheters. For example, see commonly assigned U.S. Patent Nos. 6,242,063 and 6,284,333. See also U.S. Patent Nos. 5,156,785; 6,325,780; 6,443,925 and 6,596,219. Moreover, the documents US 2001/043998 A1 and WO 01/34062 A describe medical balloons.

There remains a need in the art for materials which can be used in the manufacture of catheter assembly components which exhibit high strength, low profile and flexibility.

### SUMMARY OF THE INVENTION

The present invention relates to medical devices according to the claims employing novel microfibrillar polymer-polymer composites, and to methods of making the same.

In one aspect, the present invention relates to medical devices which are at least in part formed with a microfibrillar polymer-polymer composite wherein the microfibrillar polymer-polymer composite includes at least one first polymer having a melting temperature T₁ and at least one second polymer having a melting temperature T₂ which is greater than T₁.

The microfibrillar polymer-polymer composites may be used in any type of medical device. Specific examples of medical devices include catheter assemblies used in both intravascular procedures and non-intravascular procedures.

In another aspect, the present invention relates to a process of making a medical device according to the claims with a microfibrillar polymer-polymer composite, the method including the steps of melt blending at least one first polymer having a melting temperature T₁ and a least one second polymer having a melting temperature T₂, extruding the blend, allowing the blend to solidify, orienting the solidified polymer blend, and thermally treating or annealing of the oriented blend at a temperature T, wherein T₁<T<T₂. T of course may be virtually the same as T₁, the only criteria being that T is high enough to cause the polymer matrix material to flow. Thermal treatment of the blend may occur before or during further processing of the microfibrillar polymer-polymer composite to form a medical device such as by gas pressure molding, injection molding, etc.

The microfibrillar polymer-polymer composites according to the present application can be tailored such that the physical properties, for example, modulus, elongation, tensile strength, shore hardness, etc. are improved.

Other aspects of the invention are described in the Detailed Description and in the claims below.

### BRIEF DESCRIPTION OF THE DRAWINGS

FIG. 1 is a partial longitudinal cross-sectional view of a catheter assembly wherein the inner shaft is formed with a microfibrillar composite according to the invention.
FIG. 2 is a detailed view taken at 2 in FIG. 1.
FIGS. 3 and 4 are partial longitudinal cross-sectional views illustrating steps of a process of securing the proximal waist of a balloon to an outer catheter shaft.
FIG. 5 is a radial cross-sectional view of a structure according to the invention having strands of microfibrillar polymer-polymer composite extending therethrough.
FIG. 6 is a radial cross-sectional view of a structure according to the invention having strands of microfibrillar polymer-polymer composite extending therethrough showing through transmission laser techniques for thermal treatment.
FIG. 7 is a fragmentary perspective view of a structure according to the invention with parts cut away.
FIG. 8 is a perspective view of one embodiment of a balloon preform according to the invention.
FIG. 9 is a perspective view of an expandable medical balloon formed with a perform similar to that in FIG. 8.
FIG. 10 is typical a radial cross-sectional view along the longitudinal axis of a multilayer structure having a microfibrillar polymer-polymer composite layer.
FIG. 11 is a perspective view of a multilayer structure similar to that in FIG. 10.
FIG. 12 is a typical radial cross-sectional view along the longitudinal axis of another embodiment of a multilayer structure according to the invention having a microfibrillar polymer-polymer composite layer.
FIG. 13 is a perspective view of a multilayer structure similar to that in FIG. 12.
FIG. 14 is a typical radial cross-sectional view along the longitudinal axis of another embodiment of a multilayer structure having a microfibrillar polymer-polymer composite layer.
FIG. 15 is a perspective view of a multilayer structure similar to that shown in FIG. 14.

### DETAILED DESCRIPTIONS OF THE INVENTION

While this invention may be embodied in many different forms, there are described in detail herein specific embodiments of the invention. This description is an exemplification of the principles of the invention and is not intended to limit the invention to the particular embodiments illustrated.

The present invention relates to medical devices which at least in part are formed using microfibrillar polymer-polymer composites and to methods of making the same.

Microfibrillar polymer-polymer composites are those having a polymer matrix from at least one first lower melting polymer reinforced by microfibrils of a second higher melting polymer. Bundles of these microfibrils form the reinforcing phase.

The diameter of these bundles is often in the nanometer range and these composites may be referred to as nano-composites. The oriented microfibrils may have a diameter on the lower end from 100 to 300 nm and on the upper end of 1000 nm. A typical length to diameter ratio is 100. These ranges are intended for illustrative purposes only and not to limit the scope of the present invention. The ranges may vary.

The microfibrils may be randomly distributed throughout the polymer matrix, while exhibiting longitudinal and/or radial alignment with the longitudinal axis of the medical device within which the MFC is being employed. Furthermore, the microfibrils may exhibit chaotic orientation with alignment going anywhere in between longitudinal and radial depending for one thing on the processing employed after thermal treatment.

The MFCs employed herein may be manufactured according to a method including the steps of melt blending at least one first polymer component having a melting temperature T₁ and a least one second polymer component having a melting temperature T₂ and extruding, orientation of the blend, and thermally treating the oriented blend at a temperature T, wherein T₁<T<T₂. The thermal treatment step may occur before or during further processing of the microfibrillar polymer-polymer composite to form a medical device such as by gas pressure molding, injection molding, etc.

Orientation may be accomplished using any suitable method known in the art. One such method is by drawing of the blend including, for example, by cold drawing, continuous drawing, etc.

Melt blending may occur during extrusion.

The orientation step may be referred to as "fibrillization". During the fibrillization step, the blend of the at least two polymer components is oriented, the blend having at least one first polymer component having a lower melting temperature than at least one second higher melting polymer component. Orientation of the polymer components may be accomplished using any suitable drawing method, known in the art as described above.

As used herein, the term "isotropization" shall refer to substantial preservation of the oriented microfibrillar structure of the second higher melting polymer component. This may occur during thermal treatment of the oriented blend at a temperature T, wherein T₁ is the melting temperature of the at least one first lower melting polymer component and T₂ is the melting temperature of the at least one second higher melting polymer component and T₁ < T < T₂. During this step, the orientation of the at least one second higher melting polymer component, the microfibrillar component, is substantially maintained, while the orientation of the at least one first lower melting polymer component, the matrix component, is not. After this step, the matrix component for which orientation is not substantially maintained, may also be referred to in a variety of ways such as substantially relaxed, isotropic, etc.

Thermal treatment of the oriented blend may be accomplished using any technique known in the art. Suitable examples include, but are not limited to, laser energy, radiant energy, hot water, electron beam radiation, etc.

One method is to employ a vacuum oven.

Laser energy is advantageous because it can be readily employed for selective annealing of the oriented MFC blend.

Thus, the MFC preparation includes the main steps of heating to a temperature between the melting temperature of the two components, isothermal annealing and subsequent cooling. Suitably T₂ and T₁ have melting temperatures which are at least 10° apart from one another. The crystallization behavior of these MFCs thus depends on the thermal treatment conditions as well as on the polymers selected for the MFC.

The MFCs according to the invention exhibit novel characteristics over previously employed fibrillar structures such as those formed with LCP. While the LCP composites have reinforcing fibrils built from thermotropic rigid rod-like molecules, the MFCs are reinforced by microfibrils of more flexible thermoplastic macromolecules.

Further, while the LCP reinforcing elements are formed *in situ* during the extrusion stage, the finishing step of the MFC preparation is the generation of the matrix phase by annealing (isotropization).

Any blend of polymeric materials wherein one polymer component has a higher melting temperature than another polymer component used in the blend may be employed in the MFCs described herein. More than two polymer components may be employed as well. For example, a first and second polymer component may form the matrix, while a third polymer component forms the oriented microfibrillar structure, or a first polymer component may for the matrix, while second and third polymer components form the oriented microfibrillar structure, and so on and so forth.

Examples of suitable polymer matrix materials include, but are not limited to, polyesters such as the polyalkylene naphthalates including polyethylene terepthalate (PET) and polybutylene terephthalate (PBT); polyolefins such as polyethylene and including low (LDPE), medium (MDPE) and high density (HDPE) polyethylene and polypropylene; any of the polyamide materials including, for example, PA12, PA6, PA 66, etc.; block copolymers such as those having styrene end blocks and diene or other midblocks such as styrene-isoprene-styrene (SIS), styrene-ethylene/butylenes-styrene (SEBS); styrene-butadiene-styrene (SBS); styrene-ethylene/propylene-styrene (SEPS); styrene-isobutylene-styrene (SIBS), etc; block copolymers such as polyether-block-amide polyesters such as those sold under the tradename of PEBAX® available from Atofina; elastomeric polyesters and copolyesters including, but are not limited to, poly(ester-block ether) elastomers such as those sold under the tradename of HYTREL® available from DuPont de Nemours & Co., and those sold under the tradename of ARNITEL® available from DSM Engineering Plastics; etc. Copolymers of these materials may also be employed herein. As used herein, the term "copolymer" shall be hereinafter be used to refer to any polymer formed from two or more monomers including terpolymers and so forth.

PA12, PA6 and PA66, may also be referred to in the art as nylon 12, nylon 6 and nylon 66.

Other suitable classes of materials include, but are not limited to, polyethers, polyurethanes, polyimides, polycarbonates, copolymers and terpolymers thereof, and so forth.

In some embodiments, materials found to be particularly suitable for use as the reinforcing microfibrillar component include, but are not limited to, the polyalkylene terephthalates including PET and PBT, polyamide and polypropylene.

In some embodiments, materials found to be particularly suitable for the matrix material include, but are not limited to, LDPE, polyamide, poly(ether-block-amide) block copolymers, as well as mixtures thereof.

Some examples of suitable matrix material/microfibrillar structure component include, but are not limited to PE/PET, PA/PA, PA12/PET, PA6/PET, PA66/PET or PET/PA66 or PA66/PET, PE/PA6, PEBAX/PET, PE/PEBAX, PE/PA6, PE/PA12, etc.

Table 1 shows melting points for some of the various polymer materials:

**Table 1**

| Polymer type | Melting Temperature, Tm, (°C) |
|---|---|
| Nylon 12 | 167.5-184 |
| Nylon 6 | 193-255 |
| Nylon 66 | 211-265 (Typical dry grade 250-260) |
| LDPE (extrusion grade) | 104-113 |
| PEBA block copolymers Poly(ether-block-amide) | 134-207 |
| PET | 243-250 |
| PBT (extrusion grade) | 230 |

The above table is for illustrative purposes only. Each type of polymer is typically available in a variety of grades, where different grades may have different properties including different melting temperatures. Therefore, a range has been provided for the melting temperature of each type of polymer.

For copolymers such as poly(ether-block-amide), changing the ratio of ether to amide, may affect the melting temperature.

One criteria for selecting the matrix material and the microfibrillar polymer component is the melting temperature of the matrix material and the melting temperature of the microfibrillar polymer component which are desirably separated by a temperature span of at least 10°C, although this may vary some depending on the polymer selections. The matrix material desirably has a lower melting temperature than the microfibrillar polymer component in order to form a preferred MFC. Furthermore, each polymer component selected may in turn depend on the application to which the resultant MFC will be put. For example, the selection of the matrix and the microfibrillar component may be different for a catheter shaft, than for a catheter balloon, as well as among different balloons depending on the medical procedure for which they are to be employed. Furthermore, the amount of matrix versus the amount of microfibrillar component in the MFC, may be desirably different for the proximal end of a catheter shaft, than for the distal end of a catheter shaft, for example.

The above lists of materials are intended for illustrative purposes only, and not as a limitation on the scope of the present invention. Other polymeric materials not described herein, may be employed in the MFCs for making the medical devices described herein as well.

The matrix polymer component may be employed in amounts of 50 to 95 wt-%, suitably 50 to 90 wt-%, while the microfibril component may be employed in amounts of 1 wt-% to 60 wt-%, more suitably 5 wt-% to 50 wt-% and even more suitably 10 wt-% to 40 wt-%. These ranges are intended for illustrative purposes only. Such ranges may vary depending on the polymers selected.

The blend may be tailored as desired based on properties of modulus, tensile strength, elongation, flexibility and shore hardness.

MFCs can exhibit tensile strength and Young's modulus which can be as much as 30%-50%, or more, higher than the corresponding matrix material alone, while flexibility of the matrix material is maintained or affected at a lower rate than the strength and modulus.

Furthermore, the MFCs form the basic requirements of suppressing the incompatibility and improving adhesion between the microfibrils and the matrix polymer component.

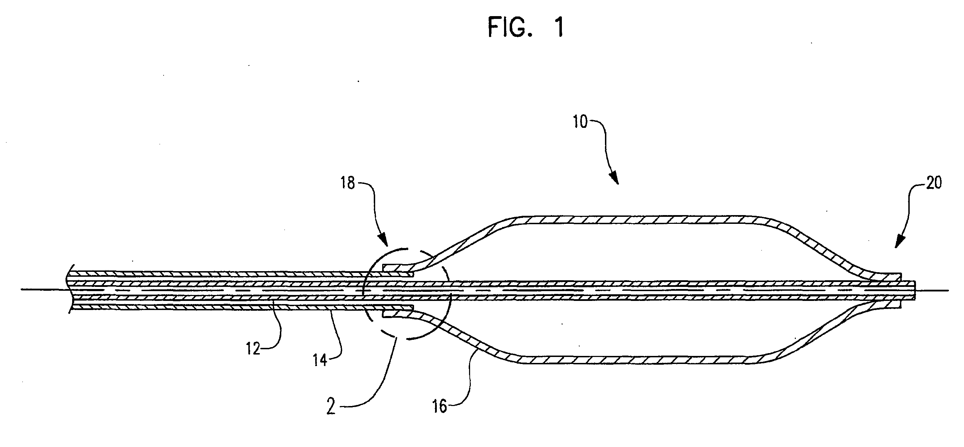

Referring now to FIG. 1, there is shown a catheter assembly 10, having an inner shaft 12, an outer shaft 14 and an expandable catheter balloon 16. Catheter balloon 16 is mounted at its distal end 20 to inner shaft 12 and at its proximal end 18 to outer shaft 14.

In this embodiment, outer shaft 14 is shown formed with a MFC according to the present application, the MFC having a first polymer component with a melting temperature T₁ and a second polymer component with a melting temperature T₂ which is higher than T₁ wherein during processing, the first polymer component according to the invention forms the matrix and the second polymer component forms the microfibrillar structure.

Any suitable polymer materials may be employed for the matrix. Examples include, but are not limited to poly(ether-block-amide) block copolymers, polyamides including PA 12 available under the tradename of Grilamid® L 25 from Ems Chemie GmbH, 50933 Cologne, DE.

Any suitable polymer material may also be employed for the microfibrillar structure. One specific example is PET.

These materials are intended for illustrative purposes only, and are not intended to limit the scope of the invention.

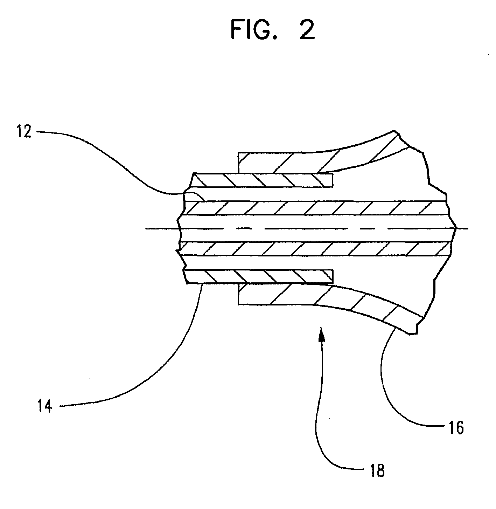

FIG. 2 is a detailed cross-sectional view of the proximal waist portion 18 of balloon 16 mounted on outer shaft 14 taken at 2 in FIG. 1.

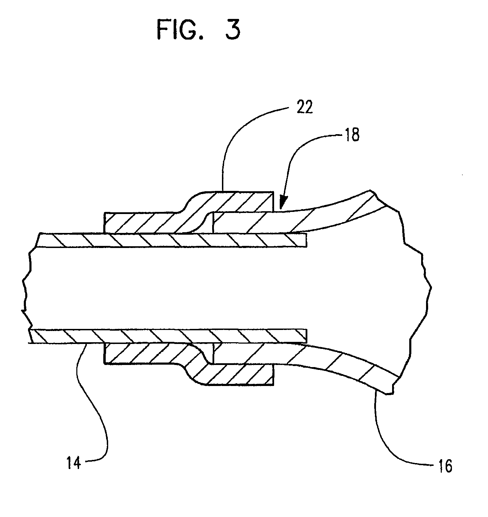

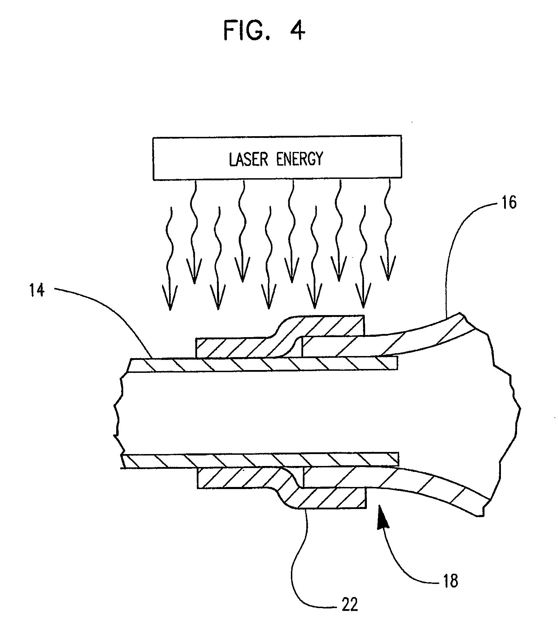

During assembly of the catheter components, proximal waist 18 of balloon 16 can be secured to distal outer shaft 14 such as by through transmission welding, the steps of which are illustrated in FIGS. 3 AND 4. In FIG. 3, a heat shrink 22 is positioned over proximal waist 18 of balloon 16 and distal end of outer shaft 14. Laser energy is then employed to weld proximal waist 18 of balloon 16 to distal outer shaft 14 as shown in FIG. 4.

A mandrel (not shown) may be employed to hold the assembly during welding of the components. The mandrel may be formed of any suitable materials known in the art such as stainless steel and ceramic, for example.

During welding, the temperature is controlled such that the outer shaft 14 reaches a temperature T which is below the melting temperature T₂ that of the second polymer component of the MFC such that this polymer component maintains the microfibrillar structure, but above the melting temperature T₁ of the first polymer component which is forming the matrix of the MFC. The matrix material is thus heated to a temperature wherein a weld can occur between the outer shaft 14 and the proximal waist 18 of the balloon.

Maintaining the microfibrillar structure within the matrix in this fashion can improve the structural integrity of the shaft. Further, the matrix material may be selected so as to provide sufficient flexibility for ease of maneuvering the assembly through tortuous bodily lumens during a medical procedure. Thus, the polymer components of the MFC can be selected so as to tailor the resultant properties of the MFC to the application to which the medical device or component thereof, is being put.

The distal waist 20 of balloon 16 may then be secured to the distal end of inner shaft 12 as shown in FIG. 1 using any methods known in the art.

It is important to note that more than one polymer material may be employed in forming the microfibrillar structure of the MFC and more than one polymer material, i.e. a blend, may be used to form the matrix of the MFC, providing that the processing temperature of the MFC is below the melting temperature of each polymer material in the MFC, and above the melting temperature of the blend which forms the matrix.

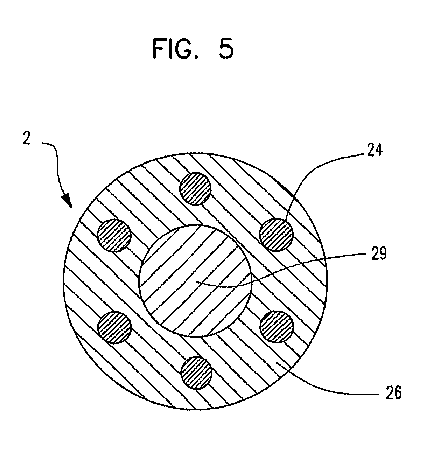

FIG. 5 is a radial cross-sectional view of another embodiment of the invention wherein a tubular member 2 has radially spaced strands 24 of MFC dispersed longitudinally in a non-MFC polymer matrix 26. Mandrel 29 extends through tubular member 2.

MFC strands 24 are formed with a blend of a first polymer component with a melting point T₁ and a second polymer component with a melting point T₂ as described above.

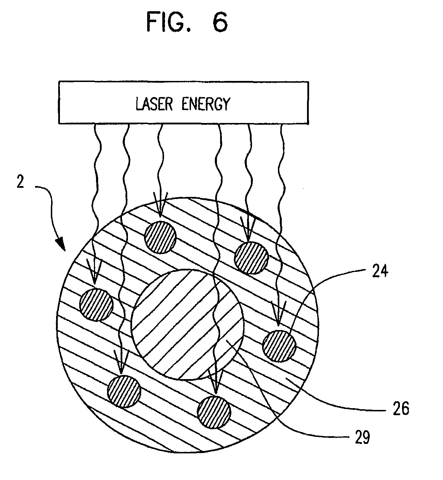

One method of dispersing the MFC strands 24 in polymer matrix 26, is by using conventional coextrusion of two polymeric materials, a technique which is well known. The entire structure may then be stretched resulting in orientation of both the MFC strands 24 and the polymer matrix 26. The MFC may then be annealed at a temperature T, wherein T₁< T < T₂. The first polymer component thus forms the matrix of the MFC and the second polymer component forms the microfibrillar structure of the MFC. Annealing of the MFC may be accomplished through the use of transmission laser techniques, for example, as shown in FIG. 6, wherein the MFC strands 24 are heated to a temperature below T₂ wherein the microfibrillar structure of the second polymer component is maintained, or the matrix material is heated at least to temperature T₁.

Another method which may be employed is to first form an MFC by extruding a blend of two polymers suitable for MFC formation, orienting the blend, allow it to cool and set, and then run the MFC strands through the extruder while extruding a non-MFC polymer material over the MFC strands. The strands may then be annealed after the tube 2 has been formed. Such methods are also known to those of skill in the art. For example, such a method is described in commonly assigned U.S. Patent No. 5,512,051.

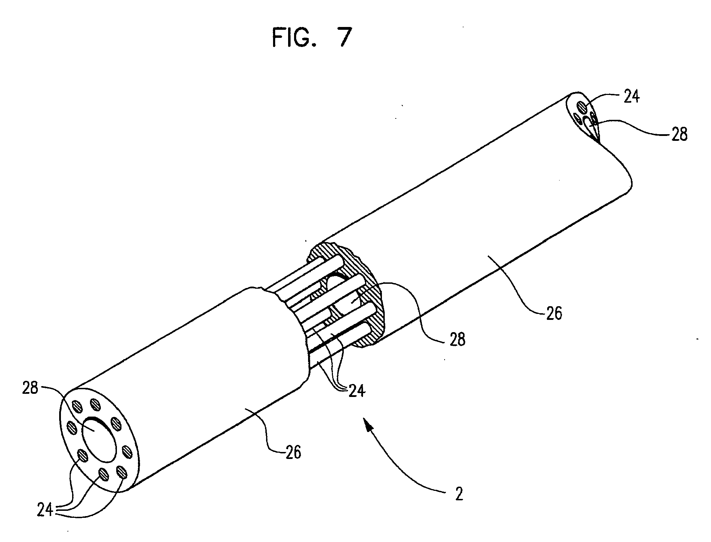

FIG. 7 is a fragmentary perspective view a structure similar to those shown in FIGS. 5 and 6 with parts cut away. After removal of the mandrel, tubular member 2 has a lumen 29 which extends therethrough.

In this embodiment, strands 24 of MFC may be disposed in the polymer matrix 26 which defines the tube wall and extend throughout the entire length of tube 2 as shown in FIG. 7, or alternatively, they may extend only partially throughout the tube wall. The resultant structure thus has MFC strands distributed in a secondary polymer matrix component which may be the same as or different than the polymer matrix component employed to form the MFC.

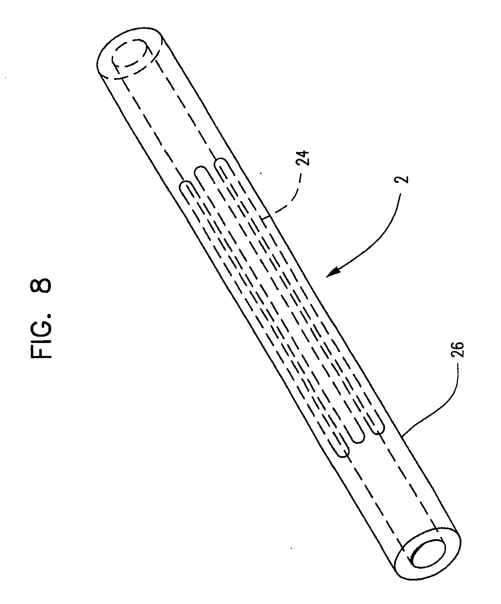

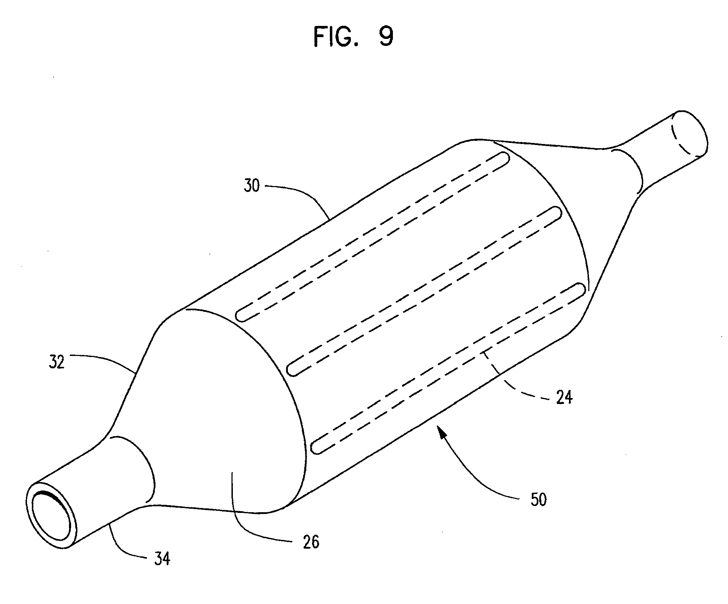

For example, in an alternative embodiment as shown in FIG. 8, the MFC strands 24 are shown provided within a polymer matrix 26 and extending through only a portion of the wall of the tube 2 is shown in FIG. 8. Such a tube may be employed as a balloon preform which may be further processed using conventional balloon forming techniques such as by gas pressure molding of a tubular extrudate. An expandable balloon member 50 formed from a tube similar to that shown in FIG. 8, is shown perspectively in FIG. 9. The MFCs strands 24 are shown extending in balloon body 30 but not in the cones 32 or waist 34 of the balloon.

The balloon may be fabricated from any suitable balloon material, typically high strength is desired, known in the art, including, but not limited to, polyethylene terephthalate (PET) and polybutylene terephthalate (PBT), polyamides (nylon), polyolefins such as polypropylene (PP), and other non-compliant engineering resins known to be suitable for medical balloon applications. Suitable compliant/semi-compliant materials are polyethylene (PE), polyvinyl chloride (PVC), PEBA (poly(ether-block amide) block copolymers) and other compliant/semi-compliant polymers known to be suitable for medical balloons.

Balloon materials may also be radially oriented if desired.

The tubular members shown in FIG. 5-8 may be employed for various types of structures in a catheter assembly including the inner shaft and outer shaft of a catheter assembly, as well as a balloon preform.

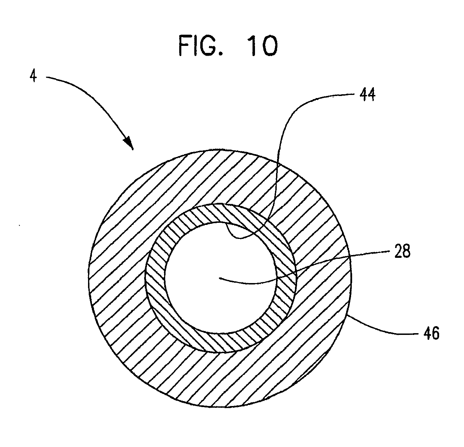

Alternatively, FIGS. 10-15 illustrate various embodiments of a tubular structure having at least one MFC layer 44 and at least one non-MFC polymer layer 46.

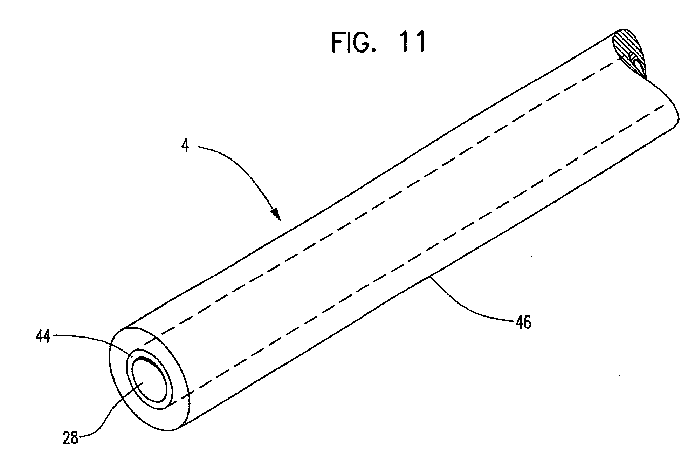

FIG. 10 is a radial cross-sectional view of a multilayer structure 4 made according to the invention which shows an inner MFC layer 44 and an outer non-MFC polymer layer 46. A similar multilayer structure 4 is shown perspectively in FIG. 11.

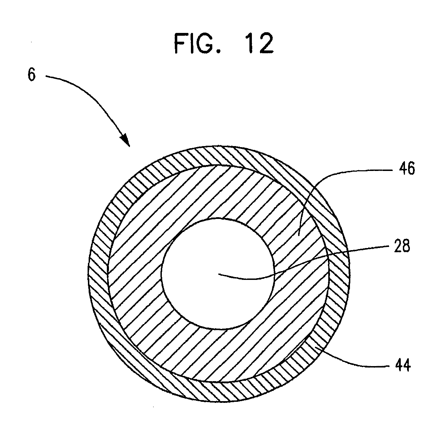

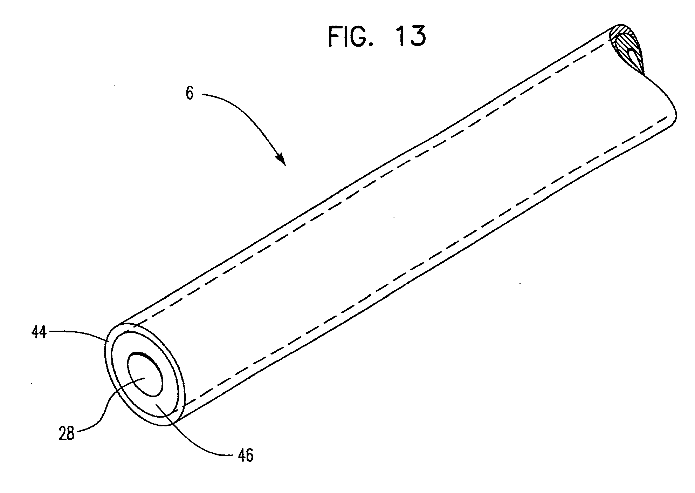

FIG. 12 is a radial cross-sectional view showing an alternative embodiment in which the MFC layer 44 forms the outer layer of a multilayer structure 6 and layer 46 of non-MFC polymer material forms the inner layer. FIG. 13 is a perspective view of a similar multilayer structure 6 to that shown in FIG. 12.

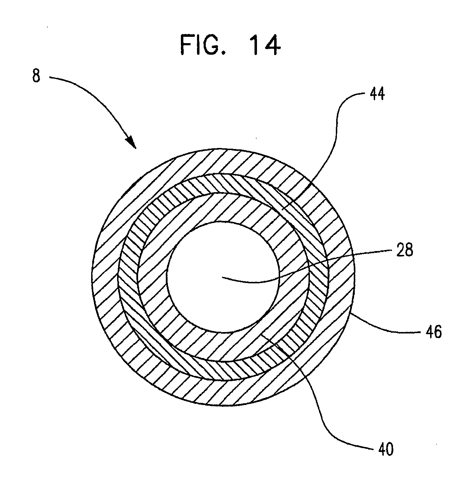

FIG. 14 is a radial cross-sectional view of a multi-layer structure 8 in which MFC layer 44 is intermediate to a non-MFC polymer layer 46 (outer layer) and a non-MFC layer 40 (inner layer).

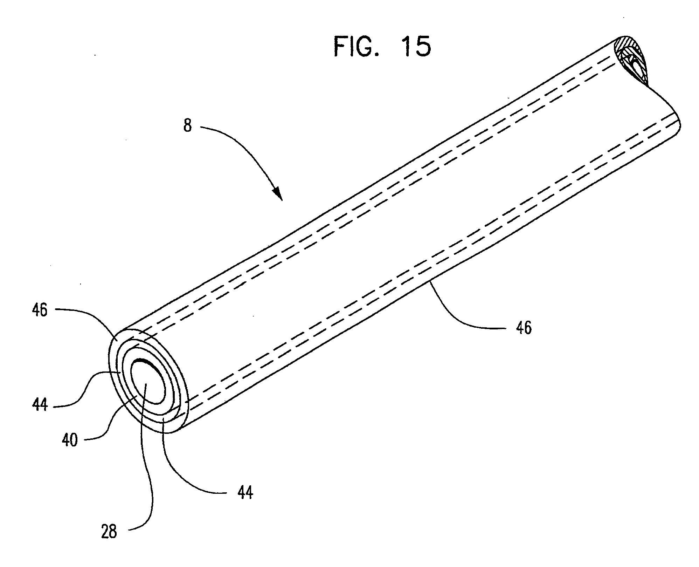

Layer 40 and layer 46 may be formed from the same polymer composition, or from a different polymer composition. A multi-layer tube similar to that shown in FIG. 14 is shown perspectively in FIG. 15.

Of course, it is also within the scope of the invention to employ a nonpolymeric layer, such as a metallic or ceramic layer, for example.

Furthermore, other types of fibers may be incorporated herein including both natural and synthetic fiber types. Examples of natural fibers include those such as silk, spider silk, wool, hemp, etc.

Examples of synthetic fibers include those formed of nylons, polyolefins such as polyethylene, liquid crystal polymers, etc.

The fibers may be incorporated in polymer layers, or fiber layers may be incorporated such as braids, weaves, roves, knits, nets, mats, etc.

The above paragraphs are intended for illustrative purposes only, and not intended to limit the scope of the present invention.

In one embodiment, the structure 8 is employed as an inner shaft 12 of a catheter assembly 10 of the type shown in FIG. 1 wherein the inner lumen 29 of structure 8 forms the guide wire lumen of the catheter assembly. In such an embodiment, it may be beneficial that layer 40 is formed from a lubricious polymer material to reduce wire movement friction through the guide wire lumen.

Lubricious polymer materials are known to those of skill in the art and include both hydrophobic and hydrophilic materials. Lubricious polymer materials are disclosed, for example, in commonly assigned U.S. Patent Nos. 6528150, 6648874, 6458867, 6444324, 6261630, 6221467, 6120904, 6017577, 5576072 and 5693034, each of which is incorporated by reference herein in its entirety. Lubricious polymer materials are also disclosed in U.S. Patent Nos. 5731087, 5645931, 5620738, 5558900, 5509899, 5509899, 5295978 and 5091205.

In the above embodiments described in FIG. 10-15, the non-MFC polymer layer may comprise any suitable polymer material depending on the application for which the tube is being employed.

Examples of suitable polymer materials include, but are not limited to, polyamides, polyethers, polyurethanes, polyesters, block copolymers having styrene end-blocks, poly(ether-block-amide) block copolymers, poly(ether-block-ester) block copolymers, poly(ester-block-ester) block copolymers, polycarbonates, polyimides, polyolefins, etc., copolymers and terpolymers thereof, and mixtures thereof.

Methods of forming layered structures are known in the art. Examples include, but are not limited to, coextrusion, overcoating, sequentially overcoating, overmolding, and so forth.

Various methods can be employed to anneal the MFC strand or layer in any of the above embodiments. Some methods may be more suitable for particular embodiments as will be discussed in more detail below.

Suitable methods of annealing the MFC layer include, but are not limited to, CO₂ lasers (IR region) and other IR lasers, diode lasers (visible spectrum), yag lasers, UV lasers, hot jaws, photon sources, radiant heat, and so forth.

If, for example, as in FIG. 12, the MFC layer 44 forms an outer layer, and the non-MFC layer 46 forms an inner layer, radiant heat may be employed to heat the outer surface, while a mandrel (not shown) having coolant flowing therethrough, may be employed over which the layers 46, 44 may be formed.

Conversely, if the MFC layer 44, forms the inner layer as shown in FIG. 10, and the non-MFC layer 46 forms the outer layer, then the mandrel may be heated, while the outer layer of the tube 4 is cooled.

For a multilayer structure of the type shown in FIG. 14, laser energy may be employed to heat the MFC layer 44 which is now an intermediate layer. For example, coloring the MFC layer 44 allows for absorption of radiation from a visible wavelength laser.

The medical devices formed with the MFCs described herein present offer many advantages over those of the prior art.

For example, catheter shafts formed using the MFCs described herein, can be formed such that they are flexible, while maintaining high tensile strength and high modulus. Flexibility and high strength in the shafts are advantageous as during medical procedures, catheters are inserted and manipulated through tortuous body lumens such as blood vessels.

Furthermore, the oriented MFC blend can be manipulated using selective thermal treatment techniques to vary the orientation of the matrix component of the MFC blend such that different portions of the medical device exhibit different degrees of flexibility, strength and modulus of elasticity, for example. This can be accomplished by changing the thermal input at different portions of the medical device.

Laser energy is advantageous for selective annealing of the oriented MFC blend. Laser pulse rate and duration of the pulse can be readily changed in order to control the amount of isotropization or de-orientation of the matrix material of the MFC blend, while maintaining substantial orientation of the microfibrillar component.

In one embodiment, selective laser annealing is employed along a catheter shaft formed from an MFC blend, the shaft having a stiff proximal end wherein both the matrix component and the microfibrillar component remain highly oriented, a stiff-to-soft transitional middle section and a soft distal end wherein the matrix component has substantially no orientation after annealing. The advantage of this process is that the relative amounts of the polymer components do not have to be altered during extrusion.

Currently, such property manipulation can be conducted through the use of exchangeable extruder outputs. Selective annealing may be combined with exchangeable extruder outputs for even more control over the properties of the resultant device to allow for even more gradual transitions in the modulus and flexibility of the shaft, for example.

Selective annealing may be employed for inner and outer catheter shafts as well as catheter tips, for balloons such as for the transition of the balloon waists, bumpers, etc.

Balloons formed with the MFCs described herein can exhibit small folded profiles, high burst pressures, and, optionally, low compliance with improved foldability, pliability, and softness, and high tensile strength.

In the formation of balloons, the present invention allows for easily combining non-compliant balloon materials, which can provide high strength and high modulus, with compliant/semi-compliant materials for flexibility.

Examples of non-compliant, semi-compliant/compliant balloon materials can be found in commonly assigned U.S. Patent Nos. 6406457, 6171278, 6146356, 5951941, 5830182 and 5556383.

The present invention allows for tailoring of physical properties, such as modulus, tensile strength, elongation and shore hardness.

The above disclosure is intended to be illustrative and not exhaustive. This description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. A medical device at least a portion of which is formed with a microfibrillar polymer-polymer composite, whereby the medical device is at least in part formed with a microfibrillar polymer-polymer composite wherein the microfibrillar polymer-polymer composite includes at least one first polymer having a melting temperature T₁ and at least one second polymer having a melting temperature T₂ which is greater than T₁, wherein microfibrillar polymer-polymer composites are those having a polymer matrix from at least one first lower melting polymer reinforced by microfibrils of a second higher melting polymer, wherein microfibrillar polymer-polymer composites are created during manufacturing by drawing (fibrillization step) followed by melting of the lower-melting component during processing (isotropization step), with preservation of the microfibrils of the higher-melting component, wherein said medical device is a catheter balloon, said catheter balloon having a body, waist portions and cone portions.

2. The medical device of claim 1, said microfibrillar polymer-polymer composite comprising at least one first polymer component having a melting temperature T₁ and at least one second polymer component having a melting temperature T₂, the microfibrillar polymer-polymer composite formed by the process of:
i) melt blending said at least one first polymer component having a melting temperature T₁ and at least one second polymer component having a melting temperature T₂ to form a polymer blend;
ii) orienting the polymer blend; and
iii) thermally treating the polymer blend at a temperature T wherein T₁<T<T₂;
wherein said at least one second polymer component forms polymer microfibrils which are distributed in said at least one first polymer component which forms the matrix of said microfibrillar polymer-polymer composite.

3. The medical device of claim 1, said medical device having a longitudinal axis, and wherein said polymer microfibrils are oriented in a direction which is parallel to the longitudinal axis of the medical device, radial to the longitudinal axis, or a combination thereof.

4. The medical device of claim 1 wherein said at least one first polymer component is a member selected from the group consisting of polyamides, block copolymers having styrene endblocks, polyether-block-amide block copolymers, poly(ether-block-ester) block copolymers, poly(ester-block-ester) block copolymers, polyethylene, and mixtures thereof.

5. The medical device of claim 1 wherein said at least one second polymer component is selected from the group consisting of polyethylene terephthalate, polybutylene terephthalate and mixtures thereof.

6. The medical device of claim 1 wherein said at least one first polymer component is polyethylene terephthalate and said at least one second polymer component is polyamide or polyethylene.

7. The medical device of claim 6 wherein said polyamide is selected from the group consisting of nylon 12, nylon 6, nylon 66 and mixtures thereof.

8. The medical device of claim 1 wherein said medical device is a catheter assembly comprising an inner shaft, an outer shaft and an expandable balloon member, at least a portion of said outer shaft is formed from said microfibrillar polymer-polymer composite.

9. The medical device of claim 1, said body of said catheter balloon comprising said microfibrillar polymer-polymer composite.

10. The medical device of claim 1 wherein said matrix of said polymer-polymer composite comprises polyamide and said microfibrillar structure comprises polyethylene terephthalate.

11. The medical device of claim 1 wherein said microfibrillar polymer-polymer composite is disposed within another polymer matrix, the polymer matrix the same as or different than the matrix of said microfibrillar polymer-polymer composite.

12. The medical device of claim 1 wherein said medical device is at least in part comprised of a multilayer structure, said multilayer structure comprising at least one layer of said microfibrillar polymer-polymer composite.

13. The medical device of claim 12, said multilayer structure comprising at least an inner layer, an intermediate layer and an outer layer, said inner layer comprising a lubricious material and at least one of said intermediate layer or said outer layer comprising said microfibrillar polymer-polymer composite.

## Patentansprüche

1. Medizinische Vorrichtung, wobei zumindest ein Teil davon mit einem Mikrofibrillen-Polymer-Polymer-Komposit ausgebildet ist, wodurch die medizinische Vorrichtung zumindest teilweise mit einem Mikrofibrillen-Polymer-Polymer-Komposit ausgebildet ist, wobei das Mikrofibrillen-Polymer-Polymer-Komposit mindestens ein erstes Polymer mit einer Schmelztemperatur T₁ und mindestens ein zweites Polymer mit einer Schmelztemperatur T₂, die größer als T₁ ist, einschließt, wobei Mikrofibrillen-Polymer-Polymer-Komposite diejenigen mit einer Polymermatrix aus mindestens einem ersten niederschmelzenden Polymer, das durch Mikrofibrillen eines zweiten höherschmelzenden Polymer verstärkt ist, sind, wobei Mikrofibrillen-Polymer-Polymer-Komposite während der Herstellung durch Ziehen (Fibrillierungsschritt), gefolgt von Schmelzen der niederschmelzenden Komponente während der Verarbeitung (Isotropisierungsschritt) unter Schützen der Mikrofibrillen der höherschmelzenden Komponente gebildet werden, wobei die medizinische Vorrichtung ein Katheterballon ist, wobei der Katheterballon einen Körper, Mittelteile und Kernteile aufweist.

2. Medizinische Vorrichtung nach Anspruch 1, wobei das Mikrofibrillen-Polymer-Polymer-Komposit mindestens eine erste Polymerkomponente mit einer Schmelztemperatur T₁ und mindestens eine zweite Polymerkomponente mit einer Schmelztemperatur T₂ umfasst, wobei das Mikrofibrillen-Polymer-Polymer-Komposit durch das folgende Verfahren gebildet wird:
i) Schmelzmischen der mindestens einen ersten Polymerkomponente mit einer Schmelztemperatur T₁ und der mindestens einen zweiten Polymerkomponente mit einer Schmelztemperatur T₂ zum Bilden einer Polymermischung;
ii) Ausrichten der Polymermischung; und
iii) Wärmebehandeln der Polymermischung bei einer Temperatur T, wobei T₁<T<T₂,
wobei die mindestens eine zweite Polymerkomponente Polymermikrofibrillen bildet, die in der mindestens einen ersten Polymerkomponente, die die Matrix des Mikrofibrillen-Polymer-Polymer-Komposits bildet, verteilt sind.

3. Medizinische Vorrichtung nach Anspruch 1, wobei die medizinische Vorrichtung eine Längsachse aufweist und wobei die Polymermikrofibrillen in einer Richtung ausgerichtet sind, die zu der Längsachse der medizinischen Vorrichtung parallel, zu Längsachse radial oder einer Kombination davon ist.

4. Medizinische Vorrichtung nach Anspruch 1, wobei die mindestens eine erste Polymerkomponente ein Vertreter ist, der ausgewählt ist aus der Gruppe, bestehend aus Polyamiden, Blockcopolymeren mit Styrolendblöcken, Polyether-Block-Amid-Blockcopolymeren, Poly(ether-block-ester)-Blockcopolymeren, Poly(ester-block-ester)-Blockcopolymeren, Polyethylen und Gemischen davon.

5. Medizinische Vorrichtung nach Anspruch 1, wobei die mindestens eine zweite Polymerkomponente ausgewählt ist aus der Gruppe, bestehend aus Polyethylenterephthalat, Polybutylenterephthalat und Gemischen davon.

6. Medizinische Vorrichtung nach Anspruch 1, wobei die mindestens eine erste Polymerkomponente Polyethylenterephthalat ist und die mindestens eine zweite Polymerkomponente Polyamid oder Polyethylen ist.

7. Medizinische Vorrichtung nach Anspruch 6, wobei das Polyamid ausgewählt ist aus der Gruppe, bestehend aus Nylon 12, Nylon 6, Nylon 66 und Gemischen davon.

8. Medizinische Vorrichtung nach Anspruch 1, wobei die medizinische Vorrichtung eine Kathetergruppe ist, die einen Innenschaft, einen Außenschaft und ein ausdehnbares Ballonelement umfasst, wobei zumindest ein Teil des Außenschafts aus dem Mikrofibrillen-Polymer-Polymer-Komposit gebildet ist.

9. Medizinische Vorrichtung nach Anspruch 1, wobei der Körper des Katheterballons das Mikrofibrillen-Polymer-Polymer-Komposit umfasst.

10. Medizinische Vorrichtung nach Anspruch 1, wobei die Matrix des Polymer-Polymer-Komposits Polyamid umfasst und die Mikrofibrillenstruktur Polyethylenterephthalat umfasst.

11. Medizinische Vorrichtung nach Anspruch 1, wobei auf dem Mikrofibrillen-Polymer-Polymer-Komposit eine andere Polymermatrix abgeschieden ist, wobei die Polymermatrix dieselbe wie oder eine andere als die Matrix des Mikrofibrillen-Polymer-Polymer-Komposits ist.

12. Medizinische Vorrichtung nach Anspruch 1, wobei die medizinische Vorrichtung zumindest teilweise aus einer mehrschichtigen Struktur zusammengesetzt ist, wobei die mehrschichtige Struktur mindestens eine Schicht aus dem Mikrofibrillen-Polymer-Polymer-Komposit umfasst.

13. Medizinische Vorrichtung nach Anspruch 12, wobei die mehrschichtige Struktur mindestens eine Innenschicht, eine Zwischenschicht und eine Außenschicht umfasst, wobei die Innenschicht ein schmieriges Material umfasst und mindestens eine der Zwischenschicht oder der Außenschicht das Mikrofibrillen-Polymer-Polymer-Komposit umfasst.

## Revendications

1. Un dispositif médical dont au moins une partie est formée par un composite polymère-polymère microfibrillaire, où le dispositif médical est au moins en partie formé par un composite polymère-polymère microfibrillaire dans lequel le composite polymère-polymère microfibrillaire comprend au moins un premier polymère présentant une température de fusion T₁ et au moins un second polymère présentant une température de fusion T₂ qui est supérieure à T₁, les composites polymère-polymère microfibrillaires étant ceux comprenant une matrice polymère provenant d'au moins un premier polymère à fusion plus basse renforcé par des microfibrilles d'un second polymère à fusion plus élevée, les composites polymère-polymère microfibrillaires étant créés pendant la fabrication par tirage (étape de formation des fibrilles) suivi de la fusion du composant à fusion plus basse pendant le traitement (étape d'isotropisation), avec préservation des microfibrilles du composant à fusion plus élevée, ledit dispositif médical étant un ballonnet de cathéter, ledit ballonnet de cathéter comportant un corps, des parties d'étranglement et des parties de cône.

2. Le dispositif médical de la revendication 1, dans lequel ledit composite polymère-polymère microfibrillaire comprend au moins un premier composant polymère présentant une température de fusion T₁ et au moins un second composant polymère présentant une température de fusion T₂, le composite polymère-polymère étant formé par le processus suivant :
i) mélange avec fusion dudit au moins un premier composant polymère présentant une température de fusion T₁ et d'au moins un second composant polymère présentant une température de fusion T₂ pour former un mélange polymère ;
ii) orientation du mélange polymère ; et
iii) traitement thermique du mélange polymère à une température T, avec T₁ < T < T₂ ;
dans lequel ledit au moins un second composant polymère forme des microfibrilles de polymère qui sont distribuées dans ledit au moins un composant polymère qui forme la matrice dudit composite polymère-polymère microfibrillaire.

3. Le dispositif médical de la revendication 1, dans lequel le dispositif médical présente un axe longitudinal, et dans lequel lesdites microfibrilles de polymère sont orientées dans une direction qui est parallèle à l'axe longitudinal du dispositif médical, qui est radiale par rapport à l'axe longitudinal, ou une combinaison des précédentes.

4. Le dispositif médical de la revendication 1, dans lequel ledit au moins un premier composant polymère est un élément sélectionné dans le groupe constitué par les polyamides, les copolymères à blocs possédant des blocs d'extrémité styrène, les copolymères à blocs polyéther-bloc-amide, les copolymères à blocs poly(éther-bloc-ester), les copolymères à blocs poly(ester-bloc-ester), le polyéthylène et les mélanges des précédents.

5. Le dispositif médical de la revendication 1, dans lequel ledit au moins un second composant polymère est choisi dans le groupe constitué par le téréphtalate de polyéthylène, le téréphtalate de polybutylène et les mélanges des précédents.

6. Le dispositif médical de la revendication 1, dans lequel ledit au moins un premier composant polymère est le téréphtalate de polyéthylène et ledit au moins un second composant polymère est le polyamide ou le polyéthylène.

7. Le dispositif médical de la revendication 6, dans lequel ledit polyamide est choisi dans le groupe constitué par le nylon 12, le nylon 6, le nylon 6.6 et les mélanges des précédents.

8. Le dispositif médical de la revendication 1, dans lequel ledit dispositif médical est un ensemble de cathéter comprenant une tige intérieure, une tige extérieure et un organe à ballonnet expansible, au moins une partie de ladite tige extérieure étant formée à partir dudit composite polymère-polymère microfibrillaire.

9. Le dispositif médical de la revendication 1, dans lequel ledit corps dudit ballonnet de cathéter comprend ledit composite polymère-polymère microfibrillaire.

10. Le dispositif médical de la revendication 1, dans lequel ladite matrice dudit composite polymère-polymère comprend du polyamide et ladite structure microfibrillaire comprend du téréphtalate de polyéthylène.

11. Le dispositif médical de la revendication 1, dans lequel ledit composite polymère-polymère microfibrillaire est disposé au sein d'une autre matrice polymère, la matrice polymère étant la même ou étant différente de la matrice dudit composant polymère-polymère microfibrillaire.

12. Le dispositif médical de la revendication 1, dans lequel ledit dispositif médical est au moins en partie formé d'une structure multicouche, ladite structure multicouche comprenant au moins une couche dudit composite polymère-polymère microfibrillaire.

13. Le dispositif médical de la revendication 12, dans lequel ladite structure multicouche comprend au moins une couche intérieure, une couche intermédiaire et une couche extérieure, ladite couche intérieure comprenant un matériau glissant et au moins l'une desdites couches intermédiaire ou couche extérieure comprenant ledit composite polymère-polymère microfibrillaire.
